(19) Europäisches Patentamt
European Patent Office
Office européen des brevets

(11) Veröffentlichungsnummer: **0 207 399 B1**

(12) # EUROPÄISCHE PATENTSCHRIFT

(45) Veröffentlichungstag der Patentschrift: **14.08.91**

(51) Int. Cl.5: **G03C 1/30**, C07D 249/08, C07D 285/12

(21) Anmeldenummer: **86108433.3**

(22) Anmeldetag: **20.06.86**

(54) **Gehärtete Bindemittelschicht.**

(30) Priorität: **29.06.85 DE 3523360**

(43) Veröffentlichungstag der Anmeldung: **07.01.87 Patentblatt 87/02**

(45) Bekanntmachung des Hinweises auf die Patenterteilung: **14.08.91 Patentblatt 91/33**

(84) Benannte Vertragsstaaten: **BE CH DE FR GB LI NL**

(56) Entgegenhaltungen: **DE-A- 1 808 683**

(73) Patentinhaber: **Agfa-Gevaert AG**
**W-5090 Leverkusen 1(DE)**

(72) Erfinder: **Öhlschläger, Hans, Dr.**
**Am Katterbach 34**
**W-5060 Bergisch-Gladbach 2(DE)**
Erfinder: **Schranz, Karl-Wilhelm, Dr.**
**Schillerstrasse 1**
**W-5068 Odenthal-Hahnenberg(DE)**
Erfinder: **Sobel, Johannes, Dr.**
**Willi-Baumeister-Strasse 9**
**W-5090 Leverkusen 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein Härtungsmittel für Proteine, sowie eine aus einem gehärteten Protein bestehende Bindemittelschicht und speziell ein lichtempfindliches fotografisches Aufzeichnungsmaterial mit mindestens einer Silberhalogenidemulsionsschicht, dessen Bindemittelschichten mit einer Vinylsulfonylgruppen enthaltenden heteroaromatischen Verbindung gehärtet worden sind.

Als Härtungsmittel für Proteine und im besonderen für Gelatine sind bereits zahlreiche Substanzen beschrieben worden. Hierzu gehören beispielsweise Metallsalze wie Chrom-, Aluminium- oder Zirkonsalze, Aldehyde und halogenhaltige Aldehydverbindungen, insbesondere Formaldehyd, Dialdehyde und Mucochlorsäure, 1,2- und 1,4-Diketone wie Cyclohexandion-1,2 und Chinone sowie Chloride oder Anhydride von mehrbasigen organischen Säuren, wie Anhydride von Tetracarbonsäuren, Verbindungen mit mehreren reaktionsfähigen Vinylgruppen wie Vinylsulfone, Acrylamide, Verbindungen mit mindestens zwei leicht spaltbaren, heterocyclischen 3-gliedrigen Ringen wie Ethylenoxid und Ethylenimin, mehrfunktionelle Methansulfonsäureester und Bis-$\alpha$-chloracylamidoverbindungen.

In neuerer Zeit wurden hochmolekulare Härtungsmittel, wie z.B.Polyacrolein bzw. seine Derivate oder Mischpolymerisate sowie Alginsäurederivate bekannt, die speziell als schichtbegrenzte Härtungsmittel Verwendung finden.

Die Verwendung der genannten Verbindungen für fotografische Zwecke ist jedoch mit einer Reihe schwerwiegender Nachteile verbunden. Einige dieser Verbindungen sind fotografisch aktiv und sind deshalb zur Härtung fotografischer Aufzeichnungsmaterialien ungeeignet, andere beeinflussen die physikalischen Eigenschaften, wie z.B. die Brüchigkeit der Gelatineschichten so nachteilig, daß sie nicht verwendet werden können. Andere wiederum verursachen Färbungen oder eine Änderung des pH-Wertes während der Härtungsreaktion. Darüber hinaus ist es für die Härtung fotografischer Schichten besonders wichtig, daß die Härtung möglichst kurze Zeit nach dem Auftrocknen ihr Maximum erreicht, damit nicht, wie beispielsweise im Falle der Mucochlorsäure oder des Formaldehyds, sich die Durchlässigkeit des zu härtenden Materials für die Entwicklerlösung fortlaufend ändert.

In gewissen Fällen haben Vernetzungsmittel für Gelatine auch hautschädigende Wirkung, wie z.B. die Ethyleniminverbindungen, so daß ihre Anwendung schon aus physiologischen Gründen nicht angebracht ist.

Es ist weiter bekannt, Trichlortriazin, Hydroxydichlortriazin und Dichloraminotriazine als Härtungsmittel zu verwenden. Nachteilig sind hierbei der verhältnismäßig hohe Dampfdruck, die Freisetzung von Salzsäure während der Härtung und die physiologische Wirkung dieser Verbindungen. Wasserlösliche Derivate, die Carboxyl- und Sulfonsäuregruppen enthalten und die durch Umsetzung von Cyanurchlorid mit einem Mol Diaminoalkyl- oder Diaminoarylsulfonsäure oder Carbonsäure erhalten werden, zeigen diese Nachteile nicht und sind deshalb in neuerer Zeit als Härtungsmittel vorgeschlagen worden. Ihre praktische Verwendbarkeit ist jedoch begrenzt, da sie sich infolge ihrer guten Löslichkeit beim Stehen in wäßrigen Lösungen zersetzen und dadurch ihre Wirksamkeit schnell einbüßen.

Es ist schließlich bei einem Härtungsmittel für fotografische gelatinehaltige Schichten sowohl aus Herstellungsals auch aus Verarbeitungsgründen von größter Bedeutung, daß auch das Einsetzen der Vernetzungsreaktion in gewissen Grenzen bestimmbar ist, beispielsweise durch Wahl der Trocknungstemperatur oder durch Wahl des pH-Wertes.

Als Härtungsmittel für fotografische Gelatineschichten bekannt sind auch Verbindungen mit 2 oder mehreren Acrylamidgruppen im Molekül, z.B. N,N',N"-Trisacryloylhexahydrotriazin oder Methylenbisacrylamid.

Die Härtungswirkung dieser Verbindungen ist nach einiger Zeit zwar gut, jedoch sind die Verbindungen in Wasser schwer löslich, was innerhalb der Schicht zu Ungleichmäßigkeiten in der Härtung führen kann.

Besondere Probleme ergeben sich bei der in zunehmendem Maße gebrauchten Schnellverarbeitung fotografischer, insbesondere farbfotografischer Aufzeichnungsmaterialien, die gesteigerte Anforderungen an die mechanischen Eigenschaften und das Quellverhalten der Aufzeichnungsmaterialien stellt. Dazu kommen die Schwierigkeiten, die sich aus der Notwendigkeit ergeben, immer dünnere fotografische Schichten herzustellen. Man hat versucht, solche Probleme durch Anwendung verschiedenartiger Härtungsmittel zu lösen. Die bekannten Härtungsmittel haben dabei entweder neue Schwierigkeiten verursacht oder sich einfach als ungeeignet erwiesen. Zu diesen Härtungsmitteln zählen die zahlreichen bekannten Vinylsulfongruppen enthaltenden Härtungsmittel, von denen Divinylsulfon (DE-C 872 153) zu den am längsten bekannten gehört. Einer Anwendung des Divinylsulfons steht seine Toxizität entgegen.

Weiter sind durch DE-C-1 100 942 aromatische Vinylsulfonverbindungen und durch DE-A-1 547 733, DE-B2-1 808 685 und DE-A 2 348 194 Vinylsulfonylalkyl-Verbindungen bekannt geworden, darunter auch solche, die einen heterocyclischen Ring enthalten.

Die bekannten Vinylsulfonverbindungen haben sich als Härtungsmittel in mehrfacher Hinsicht als

nachteilhaft erwiesen. Sie sind entweder nicht hinreichend wasserlöslich und machen besondere Maßnahmen erforderlich, um ihre Anwendung in fotografischen Gelatineschichten zu ermöglichen, oder sie beeinflussen das Trocknungsverhalten der Schichten in nachteiliger Weise. Andere dieser Verbindungen erhöhen die Viskosität der Gießzusammensetzungen so, daß die Verarbeitung der Gießzusammensetzungen zu Schichten gestört wird. Bekannte Härter das Vinylsulfontyps bewirken auch, insbesondere in farbfotografischen Aufzeichnungsmaterialien, eine Auswanderung fotografischer Zusätze von einer in die andere Schicht, was Farbänderungen und Änderungen der fotografischen Eigenschaften zur Folge hat.

Schließlich sind aus DE-A-2 635 518 als Härtungsmittel Umsetzungsprodukte bekannt, die erhalten werden bei Umsetzungen von Verbindungen mit mindestens 3 Vinylsulfonylgruppen im Molekül mit Verbindungen mit einer wasserlöslichen Gruppe und einer Gruppe, die mit einer Vinylsulfonylgruppe reagieren kann. Es entstehen dabei anionische Vinylsulfonylverbindungen.

Diese Verbindungen haben jedoch Nachteile. Sie zeigen in gelatinehaltigen fotografischen Schichten eine starke Nachhärtung, d.h. ihre optimale Wirkung setzt erst nach längerer Lagerzeit des Materials ein. Das hat zur Folge, daß mit zunehmender Dauer der Lagerung eine abnehmende Schichtquellung in Wasser auftritt und daß sich als Folge davon die sensitometrischen Daten des Materials fortlaufend ändern. Außerdem tritt nach Zusatz der bekannten Verbindungen zu gelatinehaltigen Silberhalogenidemulsionen, insbesondere bei pH-Werten um 7, mit zunehmender Digestionsdauer ein Viskositätsanstieg auf, der einen fehlerfreien Beguß nicht mehr zuläßt.

Der Erfindung liegt die Aufgabe zugrunde, ein Härtungsmittel für proteinartige Bindemittel, insbesondere fotografische hydrophile Bindemittel, vorzugsweise Gelatine bereitzustellen, das eine für diesen Zweck ausreichende Wasserlöslichkeit und eine rasch zu optimaler Wirkung kommende Härtungsreaktion zeigt und keine störende Nachhärtungswirkung aufweist.

Gegenstand der Erfindung ist eine Schicht aus einem gehärteten proteinartigen Bindemittel bzw. ein lichtempfindliches fotografisches Aufzeichnungsmaterial mit mindestens einer gehärteten gelatinehaltigen Bindemittelschicht, dadurch gekennzeichnet, daß zum Härten eine heteroaromatische Vinylsulfonverbindung mit mindestens zwei an je ein C-Atom eines 5- oder 6-gliedrigen heteroaromatischen Ringes gebundenen Vinylsulfonylgruppen verwendet worden ist.

Die erfindungsgemäß verwendeten Härtungsmittel entsprechen vorzugsweise der folgenden allgemeinen Formel I

$$Z-\left[SO_2-CH=CH_2\right]_n \qquad \text{I}$$

worin bedeuten

- Z einen gegebenenfalls substituierten heteroaromatischen Ring, der mindestens n Ring-C-Atome und mindestens ein Ring-O-, Ring-S- oder Ring-N-Atom enthält, und
- n eine ganze Zahl ≧ 2, und

die Vinylsulfonylgruppen an Ring-C-Atome gebunden sind.

Unter Heteroaromaten bzw. heteroaromatischen Ringen versteht man Heteroatome enthaltende maximal ungesättigte 5-oder 6-gliedrige Ringe, die wie Benzol ein $\pi$-Elektronensextett enthalten (vergl. H. BEYER, Lehrbuch der organischen Chemie, 18. Aufl. (1976), Seite 613). Der durch Z dargestellte heteroaromatische Ring ist beispielsweise ein Triazol-, Thiadiazol-, Oxadiazol-, Pyridin-, Pyrrol-, Chinoxalin-, Thiophen-, Furan-, Pyrimidin- oder Triazinring. Er kann außer den mindestens zwei Vinylsulfonylgruppen gegebenenfalls weitere Substituenten sowie gegebenenfalls ankondensierte Benzolringe enthalten, die ihrerseits ebenfalls substituiert sein können. Beispiele von heteroaromatischen Ringen (Z) sind im folgenden aufgeführt.

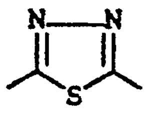 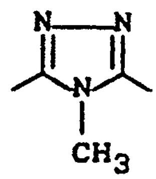 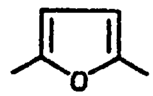

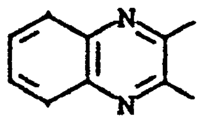 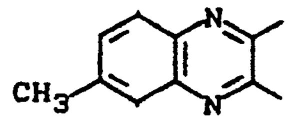

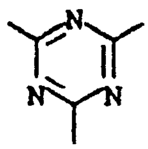 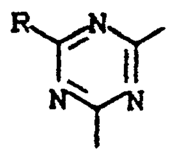

R = Alkyl,
Phenyl,
Alkoxy,

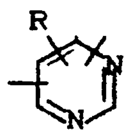

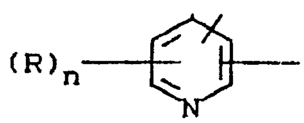

R = Alkyl,
Phenyl,
Alkoxy,
$-SO_2-CH=CH_2$
n = 0 bis 3

Beispiele erfindungsgemäßer Härtungsmittel sind im folgenden angegeben:

H-1

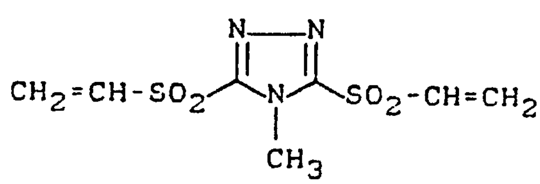

H-2

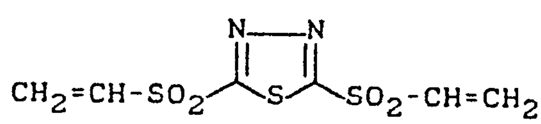

H-3

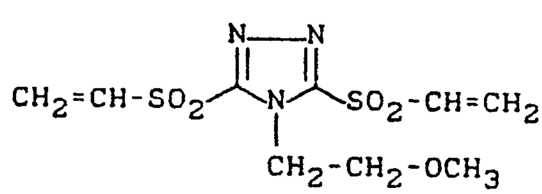

Die erfindungsgemäßen Härtungsmittel der Formel I können auf bekanntem Wege hergestellt werden, z.B. durch Umsetzung von heterocyclischen Mercaptoverbindungen $Z(SH)_n$ mit Chorethanol bzw. von heterocyclischen Polyhalogenverbindungen $Z(Hal)_n$ mit Mercaptoethanol zu den Hydroxyethylthioverbindungen $Z(S\text{-}CH_2\text{-}CH_2\text{-}OH)_n$, die in bekannter Weise mit Wasserstoffperoxid in die Hydroxyethylsulfonylderivate $Z(SO_2\text{-}CH_2CH_2\text{-}OH)_n$ überführt werden können. Diese liefern bei Umsetzung mit z.B. Thionylchlorid die Chlorethylsulfonylverbindungen $Z(SO_2\text{-}CH_2\text{-}CH_2\text{-}Cl)_n$, aus denen man durch Abspaltung von Chlorwasserstoff mit Basen wie z.B. Triethylamin die erfindungsgemäßen Härtungsmittel (I) erhält.

Im folgenden ist die Herstellung der erfindungsgemäßen Verbindung H-1 im einzelnen beschrieben, weitere Verbindungen können nach dem gleichen Schema hergestellt werden.

1. Stufe

3,5-Bis(hydroxyethylthio)-4-methyl-1,2,4-triazol

In eine Lösung von 130,8 g KOH in 1000 ml absolutem Methanol wurden 143 g 3,5-Dimercapto-4-methyl-1,2,4-triazol eingetragen, die Lösung wurde zum Rückfluß erhitzt und 188 g Chlorethanol wurden zugetropft. Die Mischung wurde noch 4,5 h bei Siedetemperatur gerührt, abgekühlt und das ausgefallene Kaliumchlorid wurde abgesaugt. Das Methanol wurde abdestilliert und der ölige Rückstand wurde mit 700 ml Acetonitril verrührt. Das auskristallsierte Produkt wurde abgesaugt und im Vakuum getrocknet. Ausbeute: 164 g = 72 %, Fp. 87-88° C.

2. Stufe

3,5-Bis-(hydroxyethylsulfonyl)-4-methyl-1,2,4-triazol

Zu einer Lösung von 101 g der unter 1. hergestellten Hydroxyethylthioverbindung in 100 ml Wasser wurden 8,8 g Phosphorsäure und 1,3 g Natriumwolframat zugesetzt und 166 g 35 %ige Wasserstoffperoxidlösung zugetropft, wobei die Temperatur durch Kühlung mit Eiswasser auf 30-35° C gehalten wurde. Die Mischung wurde noch 3 h bei Raumtemperatur nachgerührt, die auskristallisierte Substanz abgesaugt, aus Wasser umkristallisiert und im Vakuum getrocknet. Ausbeute 98 g = 76 %, Fp. 176-177° C.

3. Stufe

3,5-Bis-(chlorethylsulfonyl)-4-methyl-1,2,4-triazol

Zu einer Suspension von 97,3 g der unter 2 erhaltenen Hydroxyethylsulfonylverbindung in 300 ml Toluol werden 100 ml Thionylchlorid zugetropft, die Mischung wurde noch 30 min zum Rückfluß erhitzt, abgekühlt und das ausgefallene Produkt wurde abgesaugt und aus Aceton umkristallisiert. Ausbeute 94,5 = 85 %, Fp. 156° C.

4. Stufe

3,5-Bis-(vinylsulfonyl)-4-methyl-1,2,4-triazol

79 g der unter 3 erhaltenen Chlorethylsulfonylverbindung wurde in 400 ml Tetrahydrofuran suspendiert und innerhalb von 30 min wurden 61 ml Triethylamin zugetropft. Die Mischung wurde noch 7 h bei 50° C gerührt, abgekühlt und das auskristallisierte Triethylamin-hydrochlorid wurde abgesaugt. Nach Einengen im Vakuum wurde dar zurückbleibende Kristallbrei aus Ethanol unter Zusatz von A-Kohle umkristallsiert. Ausbeute: 50 g = 81 %, Fp. 125-126° C.

Analog erhält man durch Umsetzung von 3,5-Dimercapto-1,3,4-thiadiazol mit Chlorethanol das 3,5-Bis-(hydroxyethylthio)-1,3,4-thiadiazol, Fp. 80° C (73 %), durch dessen Oxidation mit Wasserstoffperoxid 3,5-Bis-(hydroethylsulfonyl)-1,3,4-thiadiazol, Fp. 110° C (67 %), daraus mit Thionylchlorid das 3,5-Bis-(chlorethylsulfonyl)-1,3,4-thiadiazol, Fp. 143° C (74 %.) und durch Abspaltung von Salzsäure mit Triethylamin das 3,5-Bis-(vinylsulfonyl)-1,3,4-thiadiazol, Fp. 97-99° C, (68 %).

Die erfindungsgemäße Verbindungen sind in der Lage mit aminogruppenhaltigen Verbindungen rasch zu reagieren. Aminogruppenhaltige Polymere werden hierbei vernetzt. Aus diesem Grund sind die erfindungsgemäßen heteroaromatischen Vinylsulfonylverbindungen wertvolle Härtungsmittel für die als Bindemittel häufig verwendeten Proteine, insbesondere Gelatine.

Sie weisen gegenüber den bisher gebräuchlichen Härtungsmitteln vom Vinylsulfonyl-Typ den Vorteil auf, bereits bei Zusatz in geringeren Mengen, gelatinehaltige fotografische Schichten ausreichend zu härten.

Die in der erfindungsgemäßen Weise zu verwendenden Verbindungen sind im allgemeinen in Wasser, Aceton, Methanol oder einer Mischung von Wasser mit organischen Lösungsmitteln löslich. Soweit die Löslichkeit in Wasser nur gering ist, besteht die Möglichkeit, die Verbindungen in dispergierter Form einzusetzen. Auch die Einbringung in Form eines beladenen Latex ist möglich.

Die gemäß der Erfindung verwendeten Härtungsmittel können der Gießlösung einer herzustellenden Bindemittelschicht, z.B. einer fotografischen Schicht entweder einige Zeit vor dem Beguß oder unmittelbar vor dem Beguß, zweckmäßigerweise unter Verwendung geeigneter Dosierungseinrichtungen zugesetzt werden. Die Verbindungen können auch einer Übergußlösung zugesetzt werden, die nach Herstellung der eigentlichen Bindemittelschicht als Härtungsschicht auf diese aufgebracht wird. Der fertiggestellte Schichtaufbau kann auch durch eine Lösung der Härtungsmittel gezogen werden und erhält dadurch die notwendige Menge an Härtungsmittel zugeführt. Schließlich lassen sich die erfindungsgemäßen Härtungsmittel bei Mehrschichtenaufbauten, wie sie beispielsweise bei Colorfilmen und Colorpapier gebräuchlich sind, über die Zwischenschichten in den Gesamtaufbau einbringen.

Die erfindungsgemäßen Härtungsmittel werden im allgemeinen in einer Menge von 0,01 bis 15 Gew.-% und vorzugsweise von 0,1 bis 5 Gew.-%, bezogen auf das Trockengewicht der Gelatine in der Beschichtungslösung, angewandt. Der Zeitpunkt der Zugabe zu der Beschichtungslösung ist nicht kritisch; Silberhalogenidemulsionen wird man den Härter aber zweckmäßigerweise nach der chemischen Reifung zusetzen.

Die erfindungsgemäßen Härtungsmittel können einzeln oder als Gemisch aus zwei oder mehreren erfindungsgemäßen Verbindungen, oder auch zusammen mit anderen bekannten Härtungsmitteln verwendet werden. Geeignete zusätzliche Härtungsmittel sind z.B. Formaldehyd, Glutaraldehyd und ähnliche Aldehydverbindungen, Diacetyl, Cyclopentadion und ähnliche Ketonverbindungen, Bis-(2-chlorethylharnstoff), 2-Hydroxy-4,6-dichlor-1,3,5-triazin und andere Verbindungen, die reaktives Halogen enthalten, wie in US-A-3 288 775, US-A-2 732 303, GB-A-974 723 und GB-A-1 167 207 beschrieben; Divinylsulfon, 5-Acetyl-1,3-diacryloylhexahydro-1,3,5-triazin und andere Verbindungen, die eine reaktive Olefinbindung enthalten, wie in US-A-3 635 718, US-A-3 232 763 und GB-A-994 869 beschrieben; N-Hydroxymethylphthalimid und andere N-Methylolverbindungen, wie in den US-A-2 732 316 und US-A-2 586 168 beschrieben; Isocyanate, wie in US-A-3 103 437 beschrieben; Aziridinverbindungen, wie in US-A-3 017 280 und US-A-2 983 611 beschrieben; Säurederivate, wie in US-A-2 725 294 und US-A-2 725 295 beschrieben; Verbindungen vom Carbodiimidtyp, wie in US-A-3 100 704 beschrieben; Carbomoylpyridiniumsalze wie in DE-A-22 25 230 und DE-A-24 39 551 beschrieben; Carbamoyloxypyridiniumverbindungen wie in DE-A-2 408 814 beschrieben, Verbindungen mit einer Phosphor-Halogen-Bindung wie in JP-A-113 929/83 beschrieben, N-Carbonyloximid-Verbindungen wie in JP-A- 43353/81 beschrieben, N-Sulfonyloxyimido-Verbindungen wie in US-A-4 111 926 beschrieben, Dihydrochinolinverbindungen wie in US-A 4 013 468 beschrieben, 2-Sulfonyloxypyridiniumsalze wie in JP-A-110 762/81 beschrieben, Formamidiniumsalze wie in EP-A-0 162 308 beschrieben, Verbindungen mit zwei oder mehr N-Acyloxyimino-Gruppen im Molekül wie in US-A-4 052 373 beschrieben, Epoxyverbindungen, wie in US-A-3 091 537 beschrieben; Verbindungen vom Isoxazoltyp, wie in US-A-3 321 313 und US-A-3 543 292 beschrieben; Halogencarboxyaldehyde, wie Mucochlorsäure; Dioxanderivate, wie Dihydroxydioxan und Dichlordioxan; und anorganische Härter, wie Chromalaun und Zirkonsulfat. Zusätzlich zu den obigen Härtungsmitteln können die erfindungsgemäßen Härtungsmittel zusammen mit Vorstufen der oben beschriebenen Verbindungen, wie mit Alkalimetallbisulfit-Aldehyd-Addukten, Methylolderivaten von Hydantoin und primären Fettnitroalkoholen, verwendet werden. Wenn die erfindungsgemäßen Härtungsmittel zusammen mit anderen Härtern verwendet werden, kann die Menge an erfindungsgemäßen Härtungsmitteln je nach Bedarf, abhängig von dem Ziel und der Wirkung, ausgewählt werden.

Das Protein der gehärteten Bindemittelschicht gemäß vorliegender Erfindung dient in der Regel als Bindemittel für darin dispergierte reaktive oder nicht reaktive Substanzen, z.B. Farbstoffe oder Verbindungen, die beispielsweise bei Belichtung oder einer sich daran anschließenden Verarbeitung eine Veränderung erfahren und dabei eine bestimmte Wirksamkeit entfalten. Die erfindungsgemäß gehärteten Bindemittelschichten können beispielsweise in Form farbiger Überzüge vorliegen. Das erfindungsgemäß gehärtete Protein eignet sich auch als Bindemittel für diagnostische Zwecke. So können beispielsweise trockenchemische Testmittel, auch Teststreifen genannt, mit einer erfindungsgemäß gehärteten Proteinschicht ausgestattet werden, in die die für die jeweils spezifische Nachweisreaktion erforderlichen Reagentien wie Enzyme, Coenzyme, Farbbildner und dergleichen eingelagert sind. Das gehärtete Protein kann auch in fotografischen oder fotothermografischen ein- oder mehrschichtigen Aufzeichnungsmaterialien Verwendung finden, z.B. als Bindemittel für Silberhalogenid, Farbkuppler und sonstige fotografisch wirksame Substanzen.

Unter fotografischen Schichten sollen im vorliegenden Zusammenhang ganz allgemein Schichten verstanden werden, die im Rahmen fotografischer Aufzeichnungsmaterialien Anwendung finden, beispielsweise lichtempfindliche Silberhalogenidemulsionsschichten, Schutzschichten, Filterschichten, Antihaloschichten, Rückschichten, Bildempfangsschichten oder ganz allgemein fotografische Hilfsschichten.

Als lichtempfindliche gelatinehaltige Emulsionsschichten, für deren Härtung die erfindungsgemäßen Härtungsmittel vorzüglich geeignet sind, seien beispielsweise solche Schichten genannt, die lichtempfindliche Substanzen, insbesondere Silberhalogenid, gegebenenfalls in spektral sensibilisierter Form enthalten. Solche Schichten sind üblicherweise in fotografischen Aufzeichnungsmaterialien für die verschiedensten fotografischen Schwarz-Weiß- oder Farbverfahren, wie Negativ-, Positiv-, Diffusionsübertragungs- oder Druckverfahren, enthalten. Als besonders vorteilhaft haben sich die erfindungsgemäßen Härtungsmittel für die Härtung fotografischer Schichtverbände erwiesen, die zur Durchführung farbfotografischer Prozesse bestimmt sind, z.B. solcher, die Farbkuppler oder andere farbgebende Verbindungen enthalten oder solcher, die zur Behandlung mit farbkupplerhaltigen Lösungen bestimmt sind.

Die Wirkung der erfindungsgemäßen Härtungsmittel wird durch die üblichen fotografischen Zusätze nicht beeinträchtigt. Ebenso sind die Härtungsmittel indifferent gegenüber fotografisch wirksamen Substanzen, wie wasserlöslichen oder emulgierten wasserunlöslichen Farbkomponenten, Stabilisatoren, Sensibilisatoren. Sie üben ferner keinen nachteiligen Einfluß auf die lichtempfindliche Silberhalogenidemulsion aus.

Das als lichtempfindlicher Bestandteil in den lichtempfindlichen Silberhalogenid kann als Halogenid Chlorid, Bromid und lodid bzw. Mischungen davon enthalten. Beispielsweise kann der Halogenidanteil wenigstens einer Schicht zu 0 bis 12 mol-% aus lodid, zu 0 bis 50 mol-% aus Chlorid und zu 50 bis 100 mol-% aus Bromid bestehen. Es kann sich um überwiegend kompakte Kristalle handeln, die z.B. kubisch oder oktaedrisch sind oder Übergangsformen aufweisen können. Im wesentlichen weisen sie eine Dicke von mehr als 0,2 µm auf. Das durchschnittliche Verhältnis von Durchmesser zu Dicke ist bevorzugt kleiner als 8:1, wobei gilt, daß der Durchmesser eines Kornes definiert ist als der Durchmesser eines Kreises mit einem Kreisinhalt entsprechend der projizierten Fläche des Kornes. Die Schichten können aber auch tafelförmige Silberhalogenidkristalle aufweisen, bei denen das Verhältnis von Durchmesser zu Dicke größer als 8:1 ist. Zu verweisen ist auf Research Disclosure 22 534 (Januar 1983). Bei den Emulsionen kann es sich um heterodisperse oder auch um monodisperse Emulsionen handeln, die bevorzugt eine mittlere Korngröße von 0,3 µm bis 1,2 µm aufweisen. Die Silberhalogenidkörner können auch einen geschichteten Kornaufbau aufweisen. Die Emulsionen können außer dem Silberhalogenid auch organische Silbersalze enthalten, z.B. Silberbenzotriazolat oder Silberbehenat.

Die Emulsionen können in der üblichen Weise chemisch und oder spektral sensibilisiert sein; sie können auch durch geeignete Zusätze stabilisiert sein. Geeignete chemische Sensibilisatoren, spektrale Sensibilisierungsfarbstoffe und Stabilisatoren sind beispielsweise in Research Disclosure 17643 beschrieben; verwiesen wird insbesondere auf die Kapitel III, IV und VI.

Das erfindungsgemäß hauptsächlich verwendete Bindemittel ist ein proteinartiges Bindemittel, insbesondere Gelatine. Wesentliches Merkmal dieses Bindemittels ist die Anwesenheit von funktionellen Gruppen, mit denen die Vinylsulfonylgruppen des erfindungsgemäßen Härtungsmittels reagieren können, insbesondere Aminogruppen. Das proteinartige Bindemittel kann teilweise z.B. durch partielle Acylierung modifiziert sein oder durch andere natürliche oder synthetische Bindemittel ersetzt sein, solange eine ausreichende Reaktionsfähigkeit mit dem erfindungsgemäßen Härtungsmittel erhalten bleibt. Begußhilfsmittel und Weichmacher können verwendet werden. Verwiesen wird auf Research Disclosure 17 643, insbesondere Kapitel IX, XI und XII.

Die Bindemittelschicht kann fotografisch inerte Teilchen anorganischer oder organischer Natur enthalten, z.B. als Mattierungsmittel oder als sogenannte Abstandshalter. Solche Teilchen können aus einem organischen Polymer bestehen. Verwiesen wird beispielsweise auf DE-A-33 31 542, DE-A-34 24 893, sowie auf Research Disclosure 17 643, Kapitel XVI.

Farbfotografische Aufzeichnungsmaterialen enthalten üblicherweise mindestens je eine Silberhalogenidemulsionsschicht für die Aufzeichnung von Licht jedes der drei Spektralbereiche Rot, Grün und Blau. Zu diesem Zweck sind die lichtempfindlichen Schichten in bekannter Weise durch geeignete Sensibilisierungsfarbstoffe spektral sensibilisiert. Blauempfindliche Silberhalogenidemulsionsschichten müssen nicht notwendigerweise einen Spektralsensibilisator enthalten, da für die Aufzeichnung von blauem Licht in vielen Fällen die Eigenempfindlichkeit des Silberhalogenids ausreicht.

Jede der genannten lichtempfindlichen Schichten kann aus einer einzigen Schicht bestehen oder in bekannter Weise, z.B. bei der sogenannten Doppelschichtanordnung, auch zwei oder auch mehr Silberhalogenidemulsionsteilschichten umfassen (DE-C-1 121 470). Üblicherweise sind rotempfindliche Silberhalogenidemulsionsschichten dem Schichtträger näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtempfindliche gelbe Filterschicht befindet. Es sind aber auch andere Anordnungen denkbar. Zwischen Schichten unterschiedlicher Spektralempfindlichkeit ist in der Regel eine nicht lichtempfindliche Zwischenschicht angeordnet, die Mittel zur Unterbindung der Fehldiffusion von Entwickleroxidationsprodukten enthalten kann. Falls mehrere Silberhalogenidemulsionsschichten gleicher Spektralempfindlichkeit vorhanden sind, können diese einander unmittelbar benachbart sein oder so angeordnet sein, daß sich zwischen ihnen eine lichtempfindliche Schicht mit anderer Spektralempfindlichkeit befindet (DE-A-1 958 709, DE-A-2 530 645, DE-A-2 622 922).

Farbfotografische Aufzeichnungsmaterialien zur Herstellung mehrfarbiger Bilder enthalten üblicherweise in räumlicher und spektraler Zuordnung zu den Silberhalogenidemulsionsschichten unterschiedlicher Spektralempfindlichkeit farbgebende Verbindungen, z.B. Farbkuppler, zur Erzeugung der unterschiedlichen Teilfarbenbilder Cyan, Purpur und Gelb.

Unter räumlicher Zuordnung ist dabei zu verstehen, daß der Farbkuppler sich in einer solchen räumlichen Beziehung zu der Silberhalogenidemulsionsschicht befindet, daß eine Wechselwirkung zwischen ihnen möglich ist, die eine bildgemäße Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit jeder der lichtempfindlichen Silberhalogendemulsionsschichten und die Farbe des aus dem jeweils räumlich zugeordneten Farbkuppler erzeugten Teilfarbenbildes in einer bestimmten Beziehung zueinander stehen, wobei jeder der Spektralempfindlichkeiten (Rot, Grün, Blau) eine andere Farbe des betreffenden Teilfarbenbildes (im allgemeinen z.B. die Farben Cyan, Purpur bzw. Gelb in dieser Reihenfolge) zugeordnet ist.

Jeder der unterschiedlich spektral sensibilisierten Silberhalogenidemulsionsschichten kann ein oder können auch mehrere Farbkuppler zugeordnet sein. Wenn mehrere Silberhalogenidemulsionsschichten gleicher Spektralempfindlichkeit vorhanden sind, kann jede von ihnen einen Farbkuppler enthalten, wobei diese Farbkuppler nicht notwendigerweise identisch zu sein brauchen. Sie sollen lediglich bei der Farbentwicklung wenigstens annähernd die gleiche Farbe ergeben, normalerweise eine Farbe, die komplementär ist zu der Farbe des Lichtes, für das die betreffenden Silberhalogenidemulsionsschichten überwiegend empfindlich sind.

Rotempfindlichen Silberhalogenidemulsionsschichten ist folglich bei bevorzugten Ausführungsformen mindestens ein nichtdifundierender Farbkuppler zur Erzeugung des blaugrünen Teilfarbenbildes zugeordnet, in der Regel ein Kuppler vom Phenol- oder α-Naphtholtyp. Grünempfindlichen Silberhalogenidemulsionsschichten ist mindestens ein nichtdiffundierender Farbkuppler zur Erzeugung des purpurnen Teilfarbenbildes zugeordnet, wobei üblicherweise Farbkuppler vom Typ des 5-Pyrazolons, des Indazolons oder des Pyrazoloazols Verwendung finden. Blauempfindlichen Silberhalogenidemulsionsschichten schließlich ist mindestens ein nichtdiffundierender Farbkuppler zur Erzeugung des gelben Teilfarbenbildes zugeordnet, in der Regel ein Farbkuppler mit einer offenkettigen Ketomethylengruppierung. Farbkuppler dieser Art sind in großer Zahl bekannt und in einer Vielzahl von Patentschriften beschrieben.

Beispielhaft sei hier auf die Veröffentlichungen "Farbkuppler" von W. PELZ in "Mitteilungen aus den Forschungslaboratorien der Agfa, Leverkusen/München", Band III, Seite 111 (1961) und von K. VENKATARAMAN in "The Chemistry of Synthetic Dyes", Vol. 4, 341 bis 387, Academic Press (1971), verwiesen, sowie auf Research Disclosure 17 643, Kapitel VIII.

Bei den Farbkupplern kann es sich sowohl um übliche 4-Äquivalentkuppler handeln als auch um 2-Äquivalentkuppler, bei denen zur Farberzeugung eine geringere Menge Silberhalogenid erforderlich ist, 2-Äquivalentkuppler leiten sich bekanntlich von den 4-Äquivalentkupplern dadurch ab, daß sie in der Kupplungsstelle einen Substituenten enthalten, der bei der Kupplung abgespalten wird, Zu den 2-Äquivalentkupplern sind sowohl solche zu rechnen, die praktisch farblos sind, als auch solche, die eine intensive Eigenfarbe aufweisen, die bei der Farbkupplung verschwindet bzw. durch die Farbe des erzeugten Bildfarbstoffes ersetzt wird. Letztere Kuppler können ebenfalls zusätzlich in den lichtempfindlichen Silberhalogenidemulsionsschichten vorhanden sein und dort als Maskenkuppler zur Kompensierung der unerwünschten Nebendichten der Bildfarbstoffe dienen, Zu den 2-Äquivalentkupplern sind aber auch die bekannten Weißkuppler zu rechnen, die jedoch bei Reaktion mit Farbentwickleroxidationsprodukten keinen Farbstoff ergeben. Zu den 2-Äquivalentkupplern sind ferner solche Kuppler zu rechnen, die in der Kupplungsstelle einen abspaltbaren Rest enthalten, der bei Reaktion mit Farbentwickleroxidationsprodukten in Freiheit gesetzt wird, und dabei entweder direkt, oder nachdem aus dem abgespaltenen Rest eine oder mehrere Gruppe abgespaltet worden sind (z.B. DE-A-27 03 145, DE-A-28 55 697, DE-A-31 05 026, DE-A-33 19 428), eine bestimmte erwünschte fotografische Wirksamkeit entfaltet, z.B. als Entwicklungsinhibitor oder -accelerator. Beispiele für solche 2-Äquivalentkuppler sind die bekannten DIR-Kuppler wie auch DAR- bzw. FAR-Kuppler. Der abgespaltbare Rest kann auch ein Ballastrest sein, so daß bei der Reaktion mit Farbentwickleroxidationsprodukten Kupplungsprodukte z.B. Farbstoffe erhalten werden können, die diffusionsfähig sind oder zumindest eine schwache bzw. eingeschränkte Beweglichkeit aufweisen.

Unter einer schwachen bzw. eingeschränkten Beweglichkeit ist eine Beweglichkeit zu verstehen, die so bemessen ist, daß die Konturen der bei der chromogenen Entwicklung gebildeten diskreten Farbstoffflecken verlaufen und ineinander verschmiert werden. Dieses Ausmaß der Beweglichkeit ist einerseits zu unterscheiden von dem üblichen Fall der völligen Unbeweglichkeit in fotografischen Schichten, der in der herkömmlichen fotografischen Aufzeichnungsmaterialien für die Farbkuppler bzw. die daraus hergestellten Farbstoffe angestrebt wird um eine möglichst hohe Schärfe zu erzielen, und andererseits von dem Fall der völligen Beweglichkeit der Farbstoffe, der beispielsweise bei Farbdiffusionsverfahren angestrebt wird. Die letztgenannten Farbstoffe verfügen meist über mindestens eine Gruppe, die sie im alkalischen Medium löslich macht. Das Ausmaß der erfindungsgemäß angestrebten schwachen Beweglichkeit kann gesteuert werden durch Variation von Substituenten um beispielsweise die Löslichkeit im organischen Medium des Ölbildners oder die Affinität zur Bindemittelmatrix in gezielter Weise zu beeinflussen.

Hochmolekulare Farbkuppler sind beispielsweise beschrieben in DE-C-1 297 417, DE-A-24 07 569, DE-A-31 48 125, DE-A 32 17 200, DE-A-33 20 079, DE-A-33 24 932, DE-A-33 31 743, DE-A-33 40 376, EP-A-27 284, US-A-4 080 211. Die hochmolekularen Farbkuppler werden in der Regel durch Polymerisation von ethylenisch ungesättigten monomeren Farbkupplern hergestellt. Sie können aber auch durch Polyaddition oder Polykondensation erhalten werden.

Die Bindemittelschichten können Filter- und Antihalofarbstoffe enthalten, z.B. Oxonolfarbstoffe wie beschrieben in US-A-2 274 782, US-A-2 611 696, FR-A-1 290 430, GB-A 1 177 429, DE-A-1 572 256, DE-A-22 59 746, DE-A-23 21 470, US-A-3 984 246, Styrylfarbstoffe wie beschrieben in US-A-2 036 546, DE-B-1 014 430, DE-B-1- 028 425, DE-B-1 112 801, DE-B-1 104 335, Azofarbstoffe wie beschrieben in DE-B-1 103 135, DE-B-1 182 067, GB-A-1 122 298, DE-A-1 547 646, Triphenylmethanfarbstoffe wie beschrieben in DE-B-1 008 114, Anthrachinonfarbstoffe wie beschrieben in US-A-2 865 752 oder Merocyanine wie beschrieben in GB-A-1 030 392 oder US-A-4 366 221. Verwiesen wird auch auf Research Disclosure 17 643, Kapitel VIII.

Die Bindemittelschichten können UV-Absorber enthalten, gegebenenfalls auch in hochmolekularer Form. Verwiesen wird auf DE-A-35 01 722, DE-A-35 05 423, DE-A-35 31 383, EP-A-0 027 242, EP-A-0 057 160, sowie auf Research Disclosure 17 643, Kapitel VIII und Research Disclosure 18 716, insbesondere Seite 650, linke Spalte.

Die Bindemittelschichten können Farbstoffstabilisatoren enthalten, wie beschrieben in DE-A-35 01 722, EP-A-0 011 051, EP-A-0 026 742, EP-A-0 069 070, EP-A-0 098 241, EP-A-0 114 028, EP-A-0 114 029, sowie Research Disclosure 17 643, Kapitel VII, insbesondere Abschnitt J.

Die Bindemittelschichten können optische Aufheller oder Weißtöner enthalten. Verwiesen wird beispielsweise auf Research Disclosure 17 643, Kapitel V.

Die Bindemittelschichten können sogenannte Scavenger-Verbindungen enthalten, d.h. Verbindungen die mit Entwickleroxidationsprodukten zu reagieren un diese an der Diffusion in Nachbarschichten zu hindern vermögen. Verwiesen wird beispielsweise auf EP-A-0 098 072, EP-A-0 124 877, EP-A-0 069 070, US-A-4 366 226 und EP-A-0 125 522, sowie auf Research Disclosure 17 643, insbesondere Kapitel VII, Abschnitt I, Resarch Disclosure 17 842 (Februar 1979) und Research Disclosure 18 716 (November 1979) insbesondere Seite 650.

Die Zugabe der einzubringenden Verbindungen kann in der Weise erfolgen, daß zunächst von der betreffenden Verbindung eineLösung oder ein Dispergat hergestellt und dann der Gießlösung zugefügt wird, Das Lösungs- oder Dispergiermittel richtet sich nach den jeweiligen Bedarf. Hydrophobe Verbindungen können unter Verwendung von hochsiedenden Lösungsmitteln, sogenannten Ölbildnern, in die Gießlösung eingebracht werden. Entsprechende Methoden sind beispielsweise beschrieben in US-A-2 322 027, DE-A-1 722 192 und EP-A-0 043 037. Die Verbindungen können auch in Form beladener Latices in die Gießlösung eingebracht werden. Verwiesen wird beispielsweise auf DE-A-25 41 230, DE-A-25 41 274, DE-A-28 35 856, EP-A-0 014 921, EP-A-0 069 671, EP-A-0 130 115, US-A-4 291 113.

Die Bindemittelschichten können des weiteren Mittel enthalten, die mit Aldehyden, insbesondere mit Formaldehyd zu reagieren vermögen, sogenannte Aldehydentfernungsmittel, oder Verbindungen, die andere eingelagerte Verbindungen, insbesondere andere Farbkuppler gegen den schädlichen Einfluß von Aldehyden zu schützen vermögen. Zu solchen Aldehydentfernungsmitteln gehören beispielsweise N,N'-Ethylenharnstoff, 2,3-Dihydroxynaphthalin oder Dimedon. Verwiesen wird beispielsweise auf DE-A-1 772 816.

Zur Verarbeitung wird das erfindungsgemäße Aufzeichnungsmaterial mit einer Farbentwicklerverbindung entwickelt. Als Farbentwicklerverbindung lassen sich sämtliche Entwicklerverbindungen verwenden, die die Fähigkeit besitzen in Form ihres Oxidationsproduktes mit Farbkupplern zu Azomethin- bzw. Indochinonfarbstoffen zu reagieren. Geeignete Farbentwicklerverbindungen sind aromatische mindestens eine primäre Aminogruppe enthaltende Verbindungen vom p-Phenylendiamintyp, beispielsweise N,N-Dialkyl-p-phenylendiamine, wie N,N-Diethyl-p-phenylendiamin, 1-(N-ethyl-N-methylsulfonamidoethyl)-3-methyl-p-phenylendiamin, 1-(N-ethyl-N-hydroxyethyl-3-methyl-p-phenylendiamin und 1(N-ethyl-N-methoxyethyl)-3-methyl-p-phenylendiamin. Weitere brauchbare Farbentwickler sind beispielsweise beschrieben in J. Amer. Chem. Soc. 73, 3106 (1951) und in G. Haist, Modern Photographie Processing, 1979, John Wiley and Sons, New York, Seite 545 ff.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$ -Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren, insbesondere z.B. Ethylendiamintetraessigsäure, Nitrilotriessigsäure, Iminodiessigsäure, N-Hydroxyethylethylendiamintriessigsäure, Alkyliminodicarbonsäuren und von entsprechenden Phosphonsäuren. Geeignet als Bleichmittel sind weiterhin Persulfate.

Günstige Ergebnisse können erhalten werden bei Verwendung eines wäßrigen Schlußbades, das keinen oder nur wenig Formaldehyd enthält.

## Beispiel 1

Auf einen mit einer Haftschicht versehenen Cellulosetriacetat-Schichtträger wurden nacheinander folgende Schichten aufgetragen (die angegebenen Mengen beziehen sich auf 1 $m^2$).

1. Eine Lichthofschutzschicht mit 4 g Gelatine und 0,7 g kolloidalem schwarzen Silber,
2. eine 6 µm dicke rotempfindliche Schicht mit 35 mmol Silberbromidiodid (5 mol-% AgI), 4 mmol eines Blaugrünkupplers der Formel

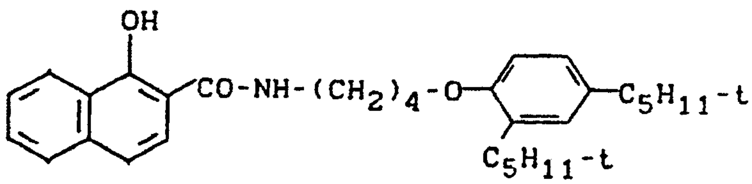

und 6 g Gelatine,

3. eine 0,5 µm dicke Gelatinezwischenschicht,
4. eine 6 µm dicke grünempfindliche Schicht mit 30 mmol Silberbromidiodid (5 mol % AgI), 1,3 mmol eines Purpurkupplers der Formel

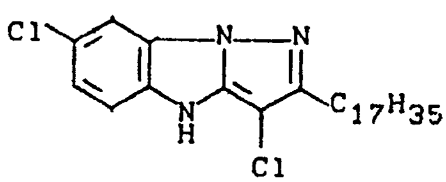

und 5 g Gelatine,

5. eine 0,5 µm diche Gelatinezwischenschicht,
6. eine Gelbfilterschicht mit 1,5 g Gelatine und 0,2 g kolloidalem gelben Silber,
7. eine 6 µm dicke blauempfindliche Schicht mit 13 mmol Silberbromidiodid (5 mol-% AgI), 2 mmol eines Gelbkupplers der Formel

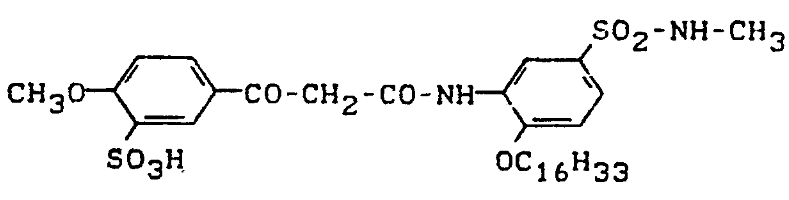

und 5 g Gelatine und

8. eine 1 µm dicke Gelatineschicht.

Der Schichtverband wurde danach getrocknet.

Der so hergestellte fotografische Film diente im folgenden als Vergleichsmaterial.

Die Herstellung des Films wurde wiederholt, wobei den einzelnen Schichten je Filmprobe eines der erfindungsgemäßen Härtungsmittel H-1 und H-2 bzw. zum Vergleich eines der nicht erfindungsgemäßen Härtungsmittel V-1, V-2 und V-3 in einer Menge von 0,6 Gew.-%, bezogen auf Gelatine zugesetzt wurden.

Die Formeln der zum Vergleich eingesetzten nicht erfindungsgemäßen Härtungsmittel sind nachfolgend angegeben:

V-1 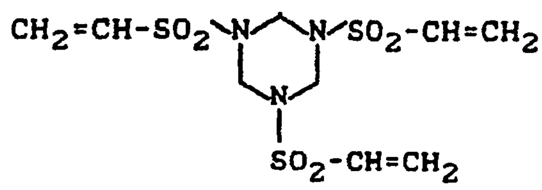

V-2 $O{\left[CH_2 - CH_2 - SO_2 - CH = CH_2\right]}_2$

V-3 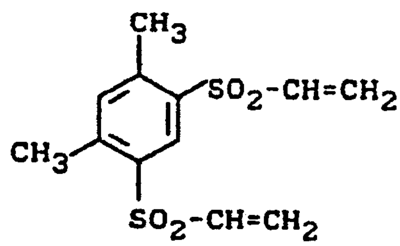

Die in der beschriebenen Weise gehärteten Proben wurden nun nach den anschließend beschriebenen Methoden geprüft. Die Ergebnisse sind in Tabelle 1 dargestellt.

Die Härtung der fotografischen Schichten wurde mit Hilfe des Schmelzpunktes der Schichten bestimmt, der sich wie folgt ermitteln läßt.

Der auf eine Unterlage vergossene Schichtverband wird zur Hälfte in Wasser getaucht, das kontinuierlich bis 100 °C erwärmt wird. Die Temperatur, bei der die Schicht von der Unterlage abläuft (Schlierenbildung), wird als Schmelzpunkt bzw. Abschmelzpunkt bezeichnet. Bei diesem Meßverfahren zeigen ungehärtete Protein- bzw. Colorschichten in keinem Falle eine Schmelzpunkterhöhung. Der Abschmelzpunkt liegt unter diesen Bedingungen bei 30 bis 35 °C.

Zur Bestimmung der Wasseraufnahme wurde der Prüfling in einem herkömmlichen Farbentwicklungsprozeß als Schwarzblatt entwickelt und nach dem Schlußbad nach Abstreifen des überschüssigen Wassers gewogen. Dann wurde die Probe getrocknet und erneut gewogen. Die Gewichtsdifferenz ergibt von der Fläche des Prüflings auf 1 $m^2$ umgerechnet die Wasseraufnahme pro $m^2$.

Die Quellung wurde nach 10 min Behandlung eines Probestreifens in destilliertem Wasser bei 22 °C gravimetrisch gemessen. Sie wird durch den Quellfaktor chrakterisiert:

$$\frac{\text{Schichtgewicht naß}}{\text{Schichtgewicht trocken}} = \text{Quellfaktor}$$

Zur Bestimmung der Naßkratzfestigkeit wurde eine Metallspitze definierter Größe über die nasse Schicht geführt und mit zunehmendem Gewicht belastet. Die Naßkratzfestigkeit wird durch das Gewicht angegeben, bei dem die Spitze eine sichtbare Kratzspur auf der Schicht hinterläßt. Ein hohes Gewicht

entspricht einer hohen Naßkratzfestigkeit.

A-G 5054

**Tabelle 1**

| Proben Nr. | Härtungsmittel | Lagerung 36 h bei 57°C, 34 % rel. LF. Schmelzpunkt der [°C] | Quellfaktor | Naßkratzfestigkeit [N] | Lagerung 7 d bei 36°C, 80 % rel. LF. Schmelzpunkt der Schicht [°C] | Quellfaktor | Naßkratzfestigkeit [N] |
|---|---|---|---|---|---|---|---|
| 1 | - | 40 | 6-8 | - | 40 | 6-8 | - |
| 2 | H-1 | 100 | 3,5 | 3,0 | 100 | 3,3 | 5,0 |
| 3 | H-2 | 100 | 3,7 | 2,5 | 100 | 3,5 | 4,5 |
| 4 | V-1 | 45 | 6-7 | < 2,0 | 95 | 5 | < 2,0 |
| 5 | V-2 | 50 | 5-6 | < 2,0 | 100 | 4,5 | < 2,5 |
| 6 | V-3 | 34 | 5-6 | < 2,0 | 100 | 5 | < 3,0 |

Es wurden keine Veränderungen der fotografischen Eigenschaften der Proben, wie Empfindlichkeit, Schleier und Gradation beobachtet. Die Quellwerte änderten sich bei Kurz- und Langzeit-Lagerung nur unwesentlich. Aus den Ergebnissen ist erkennbar, daß die erfindungsgemäßen Verbindungen eine bessere Härtungswirkung, Naßkratzfestigkeiten und Schichtschmelzpunkte als die Vergleichshärtungsmittel vom Vinylsulfontyp zeigen.

Beispiel 2

Ein Farbaufsichtsmaterial wurde hergestellt, indem auf eine polyethylen-kaschierte und mit einer Haftschicht versehenen Papierunterlage nacheinander folgende Schichten aufgetragen wurden, wobei die Emulsionsschichten die üblichen Zusätze an Netzmittel, Stabilisatoren usw. enthielten:

1. Als Unterguß eine 4 µm dicke blauempfindliche Silberhalogenidemulsionsschicht, die pro kg Emulsion 25,4 g Silberchloridbromid (12 mol-% AgCl), 80 g Gelatine und 34 g der Gelbkomponente

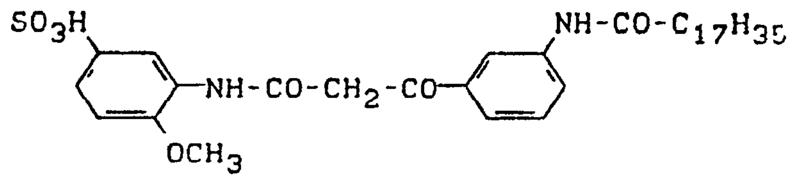

enthält.

2. als Zwischenschicht eine 1 µm dicke Gelatineschicht,

3. als Mittelguß eine 4 µm dicke grünempfindliche Silberhalogenidemulsionsschicht, die pro kg Emulsion 22 g Silberchloridbromid (77 mol-% AgCl), 80 g Gelatine und 13 g der Purpurkomponente

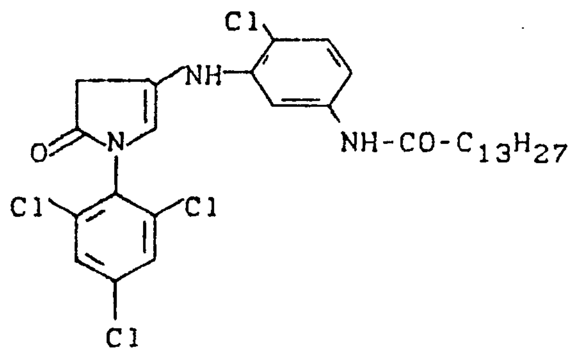

enthält,

4. eine 1 µm dicke Zwischenschicht wie unter 2. angegeben,

5. als Oberguß eine 4 µm dicke rotempfindliche Silberhalogenidemulsionsschicht, die pro kg Emulsion 23 g Silberchloridbromid (80 mol-% AgCl), 80 g Gelatine und 15,6 g der Blaugrünkomponente

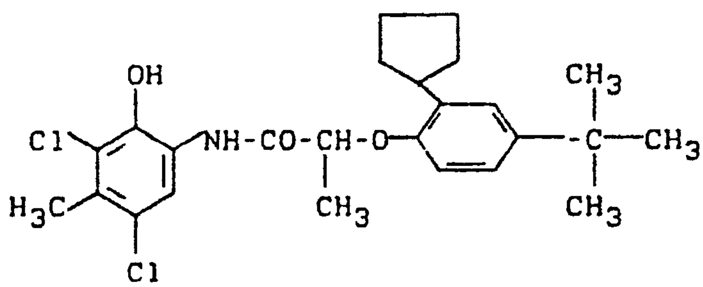

enthält,

6. eine 1 µm dicke Schutzschicht aus Gelatine.

Auf das getrocknete Schichtpaket wurden wäßrige Lösungen der Verbindungen H-1, H-2 und des nicht erfindungsgemäßen Härtungsmittels V-4 (jeweils 1/200 mol pro 100 ml) angetragen und der Schichtverband wurde danach getrocknet. Die Schichten wurden auf Vernetzung nach Klima- und Tropenlagerung untersucht.

V-4 $CH_2(-CH_2-SO_2-CH=CH_2)_2$

Die Ergebnisse sind in Tabelle 2 dargestellt.

T a b e l l e 2

| Proben Nr. | Härtungsmittel | Lagerung 36 h bei 57°C, 34 % rel. LF. Schmelzpunkt der [°C] | Quellfaktor | Naßkratzfestigkeit [N] | Lagerung 7 d bei 36°C, 80 % rel. LF. Schmelzpunkt der Schicht [°C] | Quellfaktor | Naßkratzfestigkeit [N] |
|---|---|---|---|---|---|---|---|
| 1 | H-1 | 100 | 3,7 | 2,5 | 100 | 3,4 | 5,5 |
| 2 | H-2 | 100 | 3,9 | 2,3 | 100 | 3,7 | 4,5 |
| 3 | V-4 | 40 | 5-6 | < 2,0 | 100 | 3,6 | 5,0 |

Aus dar Tabelle 2 ist ersichtlich, daß dar gesamte Schichtverband durch die eindiffundierenden erfindungsgemäßen Verbindungen bereits nach Klimalagerung kochfest gehärtet wird (im Gegensatz zur Vergleichsverbindung V-4, mit der erst nach Tropenlagerung kochfeste Schichten erhalten werden; sehr starke Nachhärtung).

Man findet bei den erfindungsgemäßen Härtern keine Abnahme der Härtungsintensität in Abhängigkeit vom Abstand von der obersten Schicht (Antragsschicht mit Härtungsmittel).

Nach der farbfotografischen Verarbeitung in den üblichen Verarbeitungsbädern werden Schichten mit vergleichbaren fotografischen Werten hinsichtlich Empfindlichkeit, Schleier, Gradation erhalten. Das Härtungssystem der Erfindung verhält sich in dieser Anwendungsform gegenüber der Emulsion und den Farbkupplern inert.

## Patentansprüche

1. Gehärtete Bindemittelschicht, bestehend aus einem Protein, das mittels einer aromatischen Vinylsulfonverbindung gehärtet ist, dadurch gekennzeichnet, daß zum Härten eine heteroaromatische Vinylsulfonverbindung mit mindestens zwei an je ein C-Atom eines 5-oder 6-gliedrigen heteroaromatischen Ringes gebundenen Vinylsulfonylgruppen verwendet worden ist.

2. Gehärtete Bindemittelschicht nach Anspruch 1, dadurch gekennzeichnet, daß zum Härten eine heteroaromatische Vinylsulfonverbindung der folgenden Formel verwendet worden ist

$$Z(-SO_2-CH=CH_2)_n$$

worin bedeuten
   Z einen gegebenenfalls substituierten heteroaromatischen Ring, der mindestens n Ring-C-Atome und mindestens ein Ring-O-, Ring-S- oder Ring-N-Atom enthält, und
   n eine ganze Zahl $\geqq 2$.

3. Gehärtete Bindemittelschicht nach Anspruch 1, dadurch gekennzeichnet, daß zum Härten 2,5-Bisvinylsulfonyl-1,3,4-thiadiazol oder 3,5-Bisvinylsulfonyl-4-methyl-1,2,4-triazol verwendet worden ist.

4. Lichtempfindliches fotografisches Aufzeichnungsmaterial mit mindestens einer mittels einer aromatischen Vinylsulfonverbindung gehärteten gelatinehaltigen Bindemittelschicht, dadurch gekennzeichnet, daß zum Härten eine heteroaromatische Vinylsulfonverbindung mit mindestens zwei an je ein C-Atome eines 5- oder 6-gliedrigen heteroaromatischen Ringes gebundenen Vinylsulfonylgruppen verwendet worden ist.

5. Aufzeichnungsmaterial nach Anspruch 4, dadurch gekennzeichnet, daß zum Härten eine heteroaromatische Vinylsulfonverbindung der folgenden Formel verwendet worden ist

$$Z(-SO_2 - CH = CH_2)_n$$

worin bedeuten
   Z einen gegebenenfalls substituierten heteroaromatischen Ring, der mindestens n Ring-C-Atome und mindestens ein Ring-O-, Ring-S- oder Ring-N-Atom enthält, und
   n eine ganze Zahl $\geqq 2$.

6. Aufzeichnungsmaterial nach Anspruch 4, dadurch gekennzeichnet, daß zum Härten 2,5-Bisvinylsulfonyl-1,3,4-thiadiazol oder 3,5-Bisvinylsulfonyl-4-methyl-1,2,4-triazol verwendet worden ist.

7. Vinylsulfonverbindung der folgenden Formel

$$Z(-SO_2 - CH = CH_2)_n$$

worin bedeuten
   Z einen gegebenenfalls substituierten 5- oder 6-gliedrigen heteroaromatischen Ring, der mindestens n Ring-C-Atome und mindestens ein Ring-O-, Ring-S- oder Ring-N-Atom enthält, und
   n eine ganze Zahl $\geqq 2$, und
wobei die Vinylsulfonylgruppen an Ring-C-Atome gebunden sind.

8. 2,5-Bisvinylsulfonyl-1,3,4-thiadiazol.

**9.** 3,5-Bisvinyl-sulfonyl-4-methyl-1,2,4-triazol

**Claims**

**1.** A hardened binder layer consisting of a protein hardened by an aromatic vinyl sulfone compound, characterized in that a heteroaromatic vinyl sulfone compound containing at least two vinyl sulfonyl groups each attached to a carbon atom of a 5- or 6-membered heteroaromatic ring has been used for hardening.

**2.** A hardened binder layer as claimed in claim 1, characterized in that a heteroaromatic vinyl sulfone compound corresponding to the following formula

$Z(-SO_2-CH=CH_2)_n$

in which
Z is an optionally substituted heteroaromatic ring containing at least n ring C atoms and at least one Ring O, ring S or ring N atom and
n is an integer of $\geqq 2$
has been used for hardening.

**3.** A hardened binder layer as claimed in claim 1, characterized in that 2,5-bis-vinylsulfonyl-1,3,4-thiadiazole or 3,5-bis-vinylsulfonyl-4-methyl-1,2,4-triazole has been used for hardening.

**4.** A photosensitive photographic recording material comprising at least one gelatine-containing binder layer hardened by an aromatic vinyl sulfone compound, characterized in that a heteroaromatic vinyl sulfone compound containing at least two vinyl sulfonyl groups each attached to a carbon atom of a 5- or 6-membered heteroaromatic ring has been used for hardening.

**5.** A recording material as claimed in claim 4, characterized in that a heteroaromatic vinyl sulfone compound corresponding to the following formula

$Z(-SO_2-CH=CH_2)_n$

in which
Z is an optionally substituted heteroaromatic ring containing at least n ring C atoms and at least one Ring O, ring S or ring N atom and
n is an integer of $\geqq 2$
has been used for hardening.

**6.** A recording material as claimed in claim 4, characterized in that 2,5-bis-vinylsulfonyl-1,3,4-thiadiazole or 3,5-bis-vinylsulfonyl-4-methyl-1,2,4-triazole has been used for hardening.

**7.** A vinyl sulfone compound corresponding to the following formula

$Z(-SO_2-CH=CH_2)_n$

in which
Z is an optionally substituted 5- or 6-membered heteroaromatic ring containing at least n ring C atoms and at least one Ring O, ring S or ring N atom and
n is an integer of $\geqq 2$,
the vinyl sulfonyl groups being attached to ring C atoms.

**8.** 2,5-Bis-vinylsulfonyl-1,3,4-thiadiazole.

**9.** 3,5-Bis-vinylsulfonyl-4-methyl-1,2,4-triazole.

**Revendications**

1. Couche de liant durcie consistant en une protéine durcie au moyen d'un composé aromatique vinylsulfonique, caractérisée en ce que, pour le durcissement, on a utilisé un composé hétéroaromatique vinylsulfonique contenant au moins deux groupes vinylsulfonyle reliés chacun à un atome de carbone d'un noyau hétéroaromatique à 5 ou 6 chaînons.

2. Couche de liant durcie selon la revendication 1, caractérisée en ce que l'on a utilisé pour le durcissement un composé vinylsulfonique hétéroaromatique répondant à la formule

$$Z(-SO_2-CH=CH_2)_n$$

dans laquelle
   Z représente un noyau hétéroaromatique éventuellement substitué contenant au moins n atomes de carbone cycliques et au moins un atome d'oxygène cyclique, un atome de soufre cyclique ou un atome d'azote cyclique, et
   n est un nombre entier supérieur ou égal à 2.

3. Couche de liant durcie selon la revendication 1, caractérisée en ce que l'on a utilisé pour le durcissement le 2,5-bis-vinylsulfonyl-1,3,4-thiadiazole ou le 3,5-bis-vinylsulfonyl-4-méthyl-1,2,4-triazole.

4. Matériau d'enregistrement photographique photosensible contenant au moins une couche de liant à base de gélatine durcie à l'aide d'un composé aromatique vinylsulfonique, caractérisé en ce que l'on a utilisé pour le durcissement un composé vinylsulfonique hétéroaromatique contenant au moins deux groupes vinylsulfonyle fixés chacun sur un atome de carbone d'un noyau hétéroaromatique à 5 ou 6 chaînons.

5. Matériau d'enregistrement selon la revendication 4, caractérisé en ce que l'on a utilisé pour le durcissement un composé hétéroaromatique vinylsulfonique répondant à la formule

$$Z(-SO_2 - CH = CH_2)n$$

dans laquelle
   Z représente un noyau hétéroaromatique éventuellement substitué contenant au moins n atomes de carbone cycliques et au moins un atome d'oxygène cyclique, un atome de soudre cyclique ou un atome d'azote cyclique, et
   n est un nombre entier supérieur ou égal à 2.

6. Matériau d'enregistrement selon la revendication 4, caractérisé en ce que l'on a utilisé pour le durcissement le 2,5-bis-vinylsulfonyl-1,3,4-thiadiazole ou le 3,5-bis-vinylsulfonyl-4-méthyl-1,2,4-triazole.

7. Composé vinylsulfonique répondant à la formule

$$Z(SO_2 - CH = CH_2)_n$$

dans laquelle
   Z représente un noyau hétéroaromatique éventuellement substitué à 5 ou 6 chaînons contenant au moins n atomes de carbone cycliques et au moins un atome d'oxygène cyclique, un atome de soufre cyclique ou un atome d'azote cylique,
   n est un nombre entier supérieur ou égal à 2, et les groupes vinylsulfonyle sont reliés à des atomes de carbone cycliques.

8. Le 2,5-bis-vinylsulfonyl-1,3,4-thiadiazole.

9. Le 3,5-bis-vinylsulfonyl-4-méthyl-1,2,4-triazole.